(19) Europäisches Patentamt
European Patent Office
Office européen des brevets

(11) Publication number : 0 632 029 A1

(12) EUROPEAN PATENT APPLICATION

(21) Application number : 94304735.7

(22) Date of filing : 29.06.94

(51) Int. Cl.[6] : C07D 233/96, A61K 31/415, A61K 7/42

(30) Priority : 30.06.93 JP 188925/93

(43) Date of publication of application : 04.01.95 Bulletin 95/01

(84) Designated Contracting States : DE FR GB IT

(71) Applicant : SHISEIDO COMPANY LIMITED
7-5-5, Ginza
Chuo-ku Tokyo 104-10 (JP)

(72) Inventor : Okazaki, Tomomi, c/o Shiseido Research Center
1050, Nippa-cho,
Kohoku-ku
Yokohama-shi, Kanagawa 223 (JP)
Inventor : Matsushita, Yuji, c/o Shiseido Research Center
1050, Nippa-cho,
Kohoku-ku
Yokohama-shi, Kanagawa 223 (JP)
Inventor : Kato, Mikiko, c/o Shiseido Research Center
1050, Nippa-cho,
Kohoku-ku
Yokohama-shi, Kanagawa 223 (JP)

(74) Representative : Perry, Robert Edward et al
GILL JENNINGS & EVERY
Broadgate House
7 Eldon Street
London EC2M 7LH (GB)

# (54) Benzylidene hydantoin derivative, ultraviolet ray absorbent and external preparation for skin including the same.

(57) A benzylidene hydantoin derivative represented by the following formula (1).

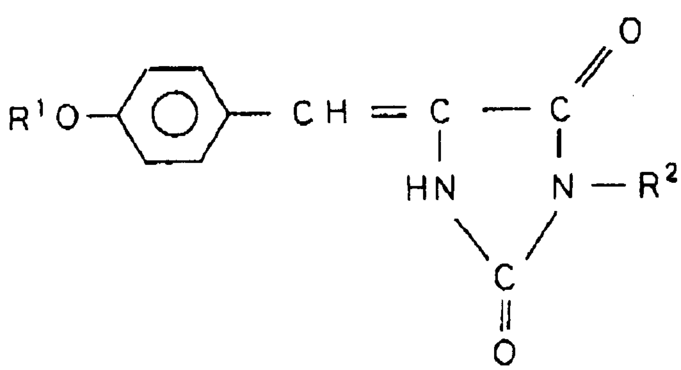

...(1)

(In the formula, $R^1$ represents a straight or branched alkyl group having 1-8 carbon atoms and $R^2$ represents a branched alkyl group having 5-18 carbon atoms.)

The derivative has excellent UV-A absorptivity and usability.

EP 0 632 029 A1

Jouve, 18, rue Saint-Denis, 75001 PARIS

SPECIFICATION

[FIELD OF THE INVENTION]

This invention relates to a benzylidene hydantoin derivative and an external preparation for skin and more particularly, a derivative having UV-A ultraviolet ray absorption, and an external preparation for skin including the same.

[BACKGROUND ART]

Various influences of ultraviolet rays to skin, such as acceleration of skin aging, have recently been well known. The ultraviolet rays included in sunlight are classified into a long wavelength ultraviolet ray having a wavelength in a range from 320 nm to 400 nm as UV-A, a medium wavelength ultraviolet ray having a wavelength in a range from 290 nm to 320 nm as UV-B, and a short wavelength ultraviolet ray having a wavelength of not more than 290 nm as UV-C in a field of skin science.

While most of the ultraviolet rays usually irradiated to a human body originate in sunlight, the UV-C is absorbed by an ozonosphere and the body is mainly influenced by the UV-A and UV-B.

Among these, the UV-B forms erythema or blister and causes aggravation of melanin formation and chromatosis when the skin is irradiated with the UV-B over a certain level.

On the other hand, the UV-A causes skin browning, decreasing of elasticity of skin, generation of wrinkles and acceleration of skin aging. Furthermore, UV-A promotes the erythema reaction and reinforces the reaction for some patients, and can be a trigger of light poison or light allergy.

In order to protect the human skin from the UV-A trouble, various UV-A absorbents have been developed.

The prior UV-A absorbents include a benzophenone derivative, a dibenzoylmethane derivative and a benzotriazol derivatives.

The UV-A absorbents generally react with a metal ion and are colored. Therefore, these have demerit of straining clothes. As a result, content of the UV-A absorbent is limited and it is impossible to present enough absorptivity of the UV-A light.

[DISCLOSURE OF THE INVENTION]

Accordingly, it is an object of the present invention to eliminate the above-described problems in the prior art and to provide a nonpolar base soluble material having a high UV-A ultraviolet ray absorptivity.

As a result of studies undertaken by the inventors so as to attain this aim, it has been found that a new benzylidene hydantoin derivative had an excellent UV-A ultraviolet ray absorptivity as well as good usability. The present invention has been achieved on the basis of this finding.

In the invention there is provided a benzylidene hydantoin derivative represented by the following formula (1).

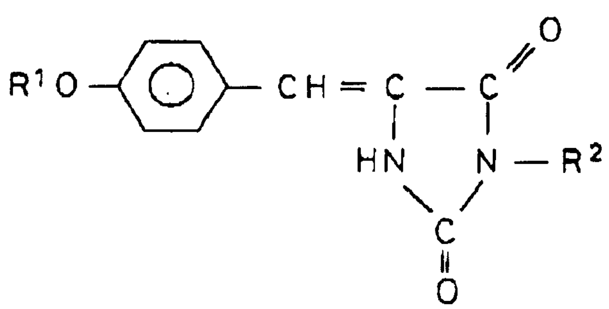

···(1)

in which $R^1$ represents a straight or branched alkyl group having 1 to 8 carbon atoms, and $R^2$ represents a branched alkyl group having 5 to 18 carbon atoms.

In the second aspect of the invention, there is provided an ultraviolet absorbent comprised of the benzylidene hydantoin derivative.

In the third aspect of the present invention, there is provided an external preparation for skin including the benzylidene hydantoin derivative.

Detailed composition of this invention will be explained hereinafter.

In the formula (1), the $R^1$ represents an alkyl group having 1 to 8 carbon atoms. The alkyl group may be

a straight chain group or a branched chain group.

For instance, the alkyl group may be a methyl group, an ethyl group, a propyl group, an isopropyl group, a butyl group, an isobutyl group, a t-butyl group, a pentyl group, an isopentyl group, a hexyl group, a 2-ethyl butyl group, a heptyl group, an octyl group or 2-ethyl hexyl group.

The $R^2$ represents a branched alkyl group having 5 to 18 carbon atoms. Although the $R^2$ may preferably be a lower alkyl group having 1 to 4 carbon atoms from a point of view of ultraviolet ray absorptivity, it is difficult to blend it in an amount sufficient to achieve the effect because of low solubility in oily base. Also, while the $R^2$ may preferably be an alkyl group having more than 19 carbon atoms from a point of view of solubility in oily base, it is difficult to achieve the effect because of low ultraviolet ray absorptivity. For instance, the $R^2$ may be an isopentyl group, a 2-ethyl butyl group, a 2-ethyl hexyl group, a 2-hexyldecyl group, a 2-heptyl undecyl group or a methyl branched isostearyl group.

In JP-A-2-111760, a benzylidene hydantoin derivative of which the $R^1$ is a methyl group and the $R^2$ is a straight alkyl group having 8 to 20 carbon atoms is disclosed. However, the prior benzylidene hydantoin derivative has low solubility in oily base and it is difficult to blend it in an amount sufficient to achieve the effect.

Also, in JP-A-2-19314, a benzylidene hydantoin derivative of which the $R^1$ is a methyl group and the $R^2$ is $-CH_2CH_2CO_2R$ is disclosed. However, the derivative has also low solubility in oily base.

On the other hand, the benzylidene hydantoin derivative according to the present invention has been improved in solubility in oily base by introducing the branched alkyl group, and can present excellent effect in ultraviolet ray absorptivity.

The benzylidene hydantoin derivative can be produced according to the following methods (a) or (b).

(a) 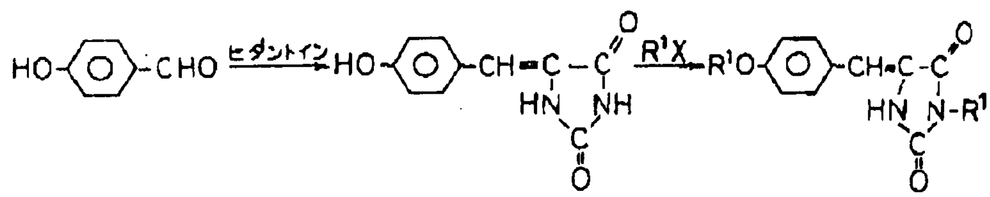


(b) 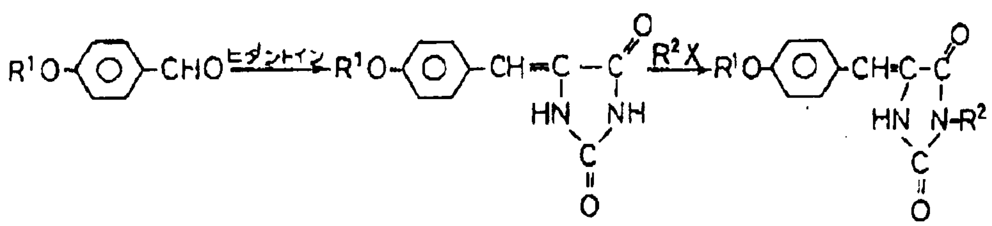


in which the $R^1X$ and $R^2X$ represent alkyl halide, respectively.

The condensation reaction of p-substituted benzaldehyde with hydantoin can be conducted according to a general condensation reaction of aldehyde with active methylene. For instance, the reaction may be a reaction described in JP-A-60-233063. The reaction may be conducted in an aqueous solvent such as water, alcohol or glycerin, or an aprotic solvent with a catalyst such as pyridine, piperidine, morpholine, aminoethanol, sodium hydroxide, potassium hydroxide, amino acid or salt of amino acid under a temperature in a range from a room temperature to 160 °C.

The alkylation reaction using alkyl halide may be conducted, for instance, in an aprotic solvent such as acetone, dimethylformamide or dimethylsulfoxide with a catalyst such as metal sodium, sodium hydroxide or potassium carbonate under a temperature in a range from 50 to 150 °C for a period in a range from 5 minutes to 8 hours.

The benzylidene hydantoin derivative according to the present invention has strong absorbance at a wavelength in a range from 320 to 370 nm and it is useful as a UV-A absorbent.

A base of the external preparation for skin according to the present invention may be any if the benzylidene hydantoin derivative can dissolve in. For instance, an oily base is preferable.

It is possible to blend any other components which can be blended in ordinal cosmetics. For instance, the components may be oily components, lubricants, antioxidants, detergents, preservatives, metal sealants, perfumes, water, alcohol or thickener.

A form of the external preparation for skin may be a powder type. a cream type, a paste type, a stick type, a liquid type, a spray type, a foundation type or an emulsified type.

Although the benzylidene hydantoin derivative can achieve enough effect itself, it is preferable to add other UV-B absorbent such a p-dimethylaminobenzoate derivative as 2-ethylhexyl p-dimethyl aminobenzoate, such

a p-methoxycinnamate derivative as neoheliopan (Harman and laymer corporation), salicylic acid derivative, urocanic acid derivative, such an inorganic pigment as titanium dioxide or zinc oxide, or other UV-A absorbent.

The content of the derivatives according to the present invention, which can be changed according to the form of the preparation or necessary ultraviolet absorptivity, is generally in a range from 0.1 to 20 weight % and more preferably 0.5 to 10 weight % in the preparation.

[EXAMPLES]

Embodiments of the present invention will be described hereinafter. It should be noted that the embodiments are not intended to limit the scope of the present invention. A unit for mixing rate is weight %.

EXAMPLE 1 PREPARATION OF 5-(4-METHOXYBENZYLIDENE)-3-(2-ETHYLHEXYL) HYDANTOIN

6.8g of p-methoxy benzaldehyde, 5.0g of hydantoin, 0.1g of sodium hydroxide and 25ml of ion exchanged water were mixed and stirred for a period of 7.5 hours at a temperature of 90 - 100°C. The mixture was then allowed to cool and precipitated residue was collected by filtration. The residue was washed with water and then methanol. As a result, 5.3g of solid mass, 4-methoxybenzylidene hydantoin, of which color was light yellow was obtained.

5.1 g of the 4-methoxybenzylidene hydantoin, so formed, 5.0g of 2-ethylhexylbromide and 3.2g of anhydrous potassium carbonate was added to 30 ml of dimethylformamide. The mixture was stirred for a period of 5 hours at a temperature of 100 - 110°C. The reactant was allowed to cool and insoluble matter was removed by filtration. The solvent of the filtrate was stripped under vacuum, and ethanol was added to the residue. The reactant was allowed to cool and crystal was precipitated The crystal, so formed, was recrystallised from ethanol and dried to give 5.8 g of colorless needle crystal.

| | |
|---|---|
| $\lambda_{max}$ (ethanol) | 334nm($\varepsilon$=32,000) |
| MAS SPECTRUM $M^+$m/e | 330 |
| MELTING POINT | 156-158°C |

EXAMPLE 2 PREPARATION OF 5-(4-METHOXYBENZYLIDENE)-3-ISOSTEARYL HYDANTOIN

4.3 g of the 4-methoxybenzylidene hydantoin, prepared according to the example 1, 5.9g of isostearylbromide, and 2.7g of anhydrous potassium carbonate were added to 20 ml of dimethylformamide and the mixture was stirred for a period of 5 hours at a temperature of 100 - 110°C. The reactant was allowed to cool and insoluble matter was removed by filtration. A solvent of the filtrate was stripped under vacuum, and then 40 ml of methanol was added to the residue. The reactant was allowed to cool and a crystal was precipitated. The crystal, so formed, was recrystallised from isopropyl alcohol and dried to give 4.5 g of the amorphous solid.

| | |
|---|---|
| $\lambda_{max}$ (ethanol) | 334nm($\varepsilon$=31,500) |
| MAS SPECTRUM $M^+$m/e | 470 |
| MELTING POINT | 100-103°C |

EXAMPLE 3 PREPARATION OF 5-[4-(2-ETHYLHEXYLOXY)BENZYLIDENE]-3-(2-ETHYLEXYL) HYDANTOIN

16.7g of p-hydroxybenzaldehyde, 13.7g of hydantoin, 6.4g of sodium hydroxide and 70ml of ion exchanged water were mixed and stirred for a period of 4 hours at a temperature of 90 - 100°C. The mixture was then allowed to cool to room temperature 500 ml of ion exchanged water was added to the mixture and adjusted pH thereof to 6 - 7 by adding hydrochloric acid. A precipitated residue was collected by filtration. The residue was washed with water and refluxed with 200 ml of ethanol for a period of 30 minutes. The system was allowed to cool. As a result, 21g of solid mass, 4-hydroxybenzylidene hydantoin was obtained.

10.2 g of the 4-hydroxybenzylidene hydantoin, so formed, 21.3g of 2-ethylhexylbromide and 13.8g of anhydrous potassium carbonate were added to 10 ml of dimethylformamide and the mixture was stirred for a period of 6 hours at a temperature of 100 - 110°C. The reactant was allowed to cool and insoluble matter was removed by filtration. A solvent of the filtrate was stripped under vacuum, 200 ml of hexane was added to the residue. After heating and stirring, insoluble matter was removed by filtration. The hexane was stripped under vacuum. The reactant was purified with a silica gel column chromatography (eluent: 10% ethylacetate-hexane) to give 11.4 g of colorless needle crystal.

| | |
|---|---|
| $\lambda_{max}$ (ethanol) | 336nm($\varepsilon$=32,500) |
| MAS SPECTRUM $M^+$m/e | 428 |
| MELTING POINT | 90-92°C |

ULTRAVIOLET RAY ABSORPTION

Ultraviolet ray absorptivities of the compound 1, 3 and comparison (2-hydroxy-4-methoxybenzophenone; UV-A absorbent) were measured according to the following method.

(Measuring Method)

The compound 1, 3 and the comparison compound were dissolved in ethanol (99.5%) so as to the concentration be 10ppm, and absorption were measured by using quartz cell (10mm×10mm) and the spectrophotometer UVIDEC-610 (JASCO CORPORATION).

The results are in the TABLE 1.

TABLE 1

| WAVELENGTH(nm) | COMPOUND 1 | COMPOUND 3 | COMPARISON |
|---|---|---|---|
| 300 | 0.36 | 0.24 | 0.39 |
| 310 | 0.53 | 0.39 | 0.35 |
| 320 | 0.74 | 0.55 | 0.40 |
| 330 | 0.95 | 0.73 | 0.42 |
| 340 | 0.94 | 0.76 | 0.34 |
| 350 | 0.80 | 0.68 | 0.20 |
| 360 | 0.40 | 0.40 | 0.11 |
| 370 | 0.10 | 0.14 | 0.03 |
| 380 | 0.02 | 0.03 | 0.00 |

As is clear from the result, the compounds according to the present invention had excellent ultraviolet ray absorptivity comparing with the comparison, and the result indicated high sun proof effect of the compounds according to the invention.

REACTION WITH METAL ION

Reactivities with trivalent iron ion of the following compounds 1 to 3 as examples of the benzylidene hydantoin derivative and 2-hydroxy-4-methoxybenzophenone (comparison 1) and 2-hydroxy-5-methylphenylbenzotriazol (comparison 2) were tested. (Method)

0.1g of each compounds were dissolved in 10 ml of 99.5% ethyl alcohol and added 2 ml of 0.02% $FeCl_3$ (99.5% ethyl alcohol solution), respectively. After stirring, color change of the solution were observed. The results are in the TABLE 2.

TABLE 2

| COMPOUND | BEFORE ADDING $FeCl_3$ | AFTER ADDING $FeCl_3$ |
|---|---|---|
| EX. 1 | COLORLESS OR LIGHT YELLOW | NO CHANGE |
| 2 | COLORLESS OR LIGHT YELLOW | NO CHANGE |
| 3 | COLORLESS OR LIGHT YELLOW | NO CHANGE |
| COM. 1 | LIGHT YELLOW | BROWNING |
| 2 | LIGHT YELLOW | BROWNING |

As is clear from the result, it is understood that the compounds according to the present invention do not change the color thereof even if with a metal ion.

SOLUBILITY

Solubilities of the compounds 1 to 3 as examples of the benzylidene hydantoin derivative and 5-(4-methoxybenzylidene)-3-n-octyl hydantoin (comparison 1, $R^1$=$CH_3$,$R^2$ =n-$C_8H_{17}$) and 5-(4-methoxybenzylidene)-3-{2-(2-ethylhexyloxycarbonyl)ethyl}hydantoin (comparison 2, $R^1$=$CH_3$, $R^2$=$CH_2CH_2CO_2C_8H_{17}$-i) were tested.

The results are in the TABLE 3.

TABLE 3

| COMPOUND | ETHANOL(99.5%) | CETYL-2-ETHYLHEXANOATE | OLIVE OIL |
|---|---|---|---|
| EX. 1 | 1.2% | 2.1% | 3.2% |
| 2 | 4.0 | 14 | 17 |
| 3 | 1.4 | 4 | 7 |
| COM. 1 | 0.65 | 0.2 | 0.4 |
| 2 | < 1 | < 1 | < 1 |

As is clear from the result, it is understood that the compounds according to the present invention have excellent solubility in nonpolar solvents. Compared the example 1 with the comparison 1, it is understood that even if the number of carbon atoms in $R^2$ are same, the example 1 is better than the comparison 1 in a point of view of solubility. It is based on the difference of $R^2$, straight or branched alkyl.

Examples of external preparations for skin including the benzylidene hydantoin derivatives will be explained hereinafter.

EXAMPLE 4 SUN SCREEN COSMETIC (OILY TYPE)

| | |
|---|---|
| (1) Decamethylcyclopentasiloxane | 47.0% |
| (2) Dimethylpolysiloxane (10cs/25℃) | 20.0 |
| (3) Methyl phenyl polysiloxane (20cs/25℃) | 18.0 |
| (4) Silicone resin | 10.0 |

(5) 2-ethylhexyl p-dimethylaminobenzoate 3.0

(6) 5-[4-(2-ethylhexyloxy)benzylidene]-3-(2-ethylhexyl)hydantoin 2.0

<Method of preparation>

<Method of preparation>

The cosmetic was prepared by mixing the ingredients (1)-(6) and filtrating. The ingredient (6) could easily dissolve in the oily system.

<Sun screen effect>

Sunscreen effects of the EXAMPLE 4, and comparative example which includes same ingredients but using 2-ethylhexyl p-dimethylamino benzoate (content : 5.0%) instead of the 5-[4-(2-ethylhexyloxy)benzylidene]-3-(2-ethylhexyl)hydantoin (ingredient (6)) were examined.

Namely, the example was applied to half sides of bodies of 10 panelers and the comparison was applied to other half sides, respectively, for the use test in the beach. And then, questionaire investigations of suntan condition and investigations of skin trouble were conducted.

The results are in the TABLE 4.

TABLE 4

| | | EXAMPLE 4 | COMPARISON |
|---|---|---|---|
| Panel | A | ○ | ○ |
| | B | ○ | × |
| | C | △ | × |
| | D | ○ | ○ |
| | E | ○ | △ |
| | F | ○ | △ |
| | G | ○ | △ |
| | H | ○ | × |
| | I | ○ | × |
| | J | ○ | × |
| Number of skin trouble; | | none | smarts : 4<br>itches : 1<br>eruption : 2 |

Evaluation standard

Strong suntan was admitted : ×

Suntan was admitted : △

Suntan was hardly admitted : ○

As is clear from the results, the ultraviolet ray shield effect of the external preparation for skin including benzylidene hydantoin derivatives were better than that of the external preparation for skin including the conventional ultraviolet absorbent (2-ethyl hexyl p-dimethylamino benzoate) and the safety thereof is also high because of none skin trouble.

EXAMPLE 5 SUNSCREEN COSMETIC (W/O CREAM)

| | |
|---|---|
| (1) Octamethylcyclotetrasiloxane | 10.5% |
| (2) Dimethylpolysiloxane (100cs) | 5.0 |
| (3) Dimethylpolysiloxane (2,500,000cs) | 3.0 |
| (4) Liquid paraffin | 15.0 |
| (5) Polyether denatured silicone | 6.0 |
| (6) 2-ethylhexyl p-dimethylamino benzoate | 5.0 |
| (7) 5-(4-isopropoxy benzylidene)-3-(2-ethylhexyl)hydantoin | 4.0 |
| (8) Purified water | 43.1 |
| (9) Monosodium L-glutamate | 3.0 |
| (10) 1, 3-butylene glycol | 5.0 |
| (11) Preservatives | 0.2 |
| (12) Perfume | 0.2 |

<Method of preparation>

An oil part was prepared by mixing, heating and dissolving the ingredients (1)-(7) and (12) and maintained the temperature thereof to 70 °C. An aqueous part was prepared by dissolving the ingredients (8)-(11) and heating at 70 °C. The aqueous part was added to the oil part and the mixture was emulsified with an emulsifier. The system was stirred and allowed to cool to a temperature of equal to or less than 35 °C. filled into a container and solidified.

EXAMPLE 6 SUNSCREEN COSMETIC (O/W CREAM)

| | |
|---|---|
| (1) Decamethylcyclopentasiloxane | 3.0 |
| (2) Liquid paraffin | 8.0 |
| (3) Isopropyl myristate | 2.0 |
| (4) Petrolatum | 4.0 |
| (5) Cetanol | 4.0 |
| (6) Stearic acid | 3.0 |
| (7) Glycerylmonoisostearate | 3.0 |
| (8) 2-ethylhexyl p-methoxycinnamate | 3.0 |
| (9) 5-(4-methoxybenzylidene)-3-(2-ethylhexyl)hydantoin | 1.0 |

| | |
|---|---|
| (10) Preservatives | 0.2 |
| (11) Perfume | 0.2 |
| (12) Glycerol | 10.0 |
| (13) Propylene glycol | 5.0 |
| (14) Hyaluronic acid | 0.01 |
| (15) Potassium hydroxide | 0.2 |
| (16) Purified water | 53.39 |

<Method of preparation>

An oil part was prepared by mixing the ingredients (1)-(11) with a disper at 70°C. An aqueous part was prepared by dissolving the ingredients (12)-(16) at 70°C. The oil part was added to the aqueous part and emulsified with an emulsifier. The emulsion was allowed to cool to 30°C with a heat exchanger, and filled in a container.

EXAMPLE 7 SUNSCREEN LOTION

| | |
|---|---|
| (1) Dimethylpolysiloxane (5cs) | 3.0% |
| (2) Methyl phenyl polysiloxane (20cs) | 17.0 |
| (3) Stearic acid | 1.0 |
| (4) 2-ethylhexyl p-dimethylaminobenzoate | 5.0 |
| (5) 5-[4-(2-ethylhexyloxy)benzylidene)-3-(2-ethylhexyl)hydantoin | 10.0 |
| (6) Preservatives | 0.2 |
| (7) Perfume | 0.2 |
| (8) Glycerol | 5.0 |
| (9) Montmorillonite | 0.5 |
| (10) Potassium hydroxide | 0.2 |
| (11) Purified water | 65.9 |

<Method of preparation>

An oil part was prepared by hand-stirring the ingredients (1)-(7) at 70 °C. An aqueous part was prepared by stirring the ingredients (8)-(11) at 70 °C. The oil part was added to the aqueous part and emulsified with an emulsifier. The emulsion was allowed to cool to 30 °C with a heat exchanger and the sunscreen lotion was obtained.

EXAMPLE 8 DUAL PURPOSE FOUNDATION

| | |
|---|---|
| (1) Silicone treated titanium oxide | 9.5% |
| (2) Silicone treated mica | 40.0 |
| (3) Silicone treated talc | 20.45 |
| (4) Silicone treated iron oxide | 7.5 |
| (5) Spherical nylon powder | 10.0 |
| (6) Trimethylolpropanetriisostearate | 5.0 |
| (7) Squalane | 3.0 |
| (8) Bess wax | 2.0 |
| (9) 5-(4-methoxybenzylidene)-3-isostearylhydantoin | 0.5 |
| (10) Sorbitan trioleate | 1.0 |
| (11) Preservatives | 0.5 |
| (12) Vitamin E | 0.05 |
| (13) Perfume | 0.5 |

<Method of preparation>

The ingredients (1)-(5) were mixed with a henchel mixer and the ingredients (6)-(13) were added therein. The product was pulverized and formed in an inner plate.

EXAMPLE 9 SUNSCREEN STICK COSMETIC

| | |
|---|---|
| (1) Titanium oxide | 10.0 |
| (2) Zinc oxide | 7.0 |
| (3) Mica | 16.0 |
| (4) Red iron oxide | 1.5 |
| (5) Yellow iron oxide | 1.5 |
| (6) Black iron oxide | 1.0 |
| (7) Dimethylpolysiloxane (20cs) | 29.4 |
| (8) Trimethylolpropane-tri-2-ethylhexanoate | 8.0 |
| (9) Liquid paraffin | 7.0 |
| (10) Microcrystalline wax | 2.0 |
| (11) Ceresin | 1.0 |
| (12) Solid paraffin | 6.0 |
| (13) 2-ethyl hexyl p-dimethylamino benzoate | 5.0 |
| (14) 5-(4-isopropoxy benzylidene)-3-(2-hexyldecyl)hydantoin | 3.0 |
| (15) Perfume | 0.5 |
| (16) Antioxidant | 0.1 |
| (17) Sorbitansesquioleate | 1.0 |

<Method of preparation>

The ingredients (1)-(6) were mixed with a henchel mixer. The ingredients (7)-(9), (13), (14), (16) and (17) were stirred, heated and dissolved, and the mixture of the ingredients (1)-(6) was added therein. A dissolved mixture of the ingredients (10)-(12) and (15) was added to the above mixture and formed stick shape.

EXAMPLE 10 SUNSCREEN COSMETIC BASE

(1) Squalane 19.0

| | |
|---|---|
| (2) Glyceryltriisostearate | 10.0 |
| (3) ISOPER G | 5.0 |
| (4) Sorbitan sesquioleate | 1.0 |
| (5) Polysiloxane ethylene denatured organopolysiloxane | 3.0 |
| (6) Purified water | 45.0 |
| (7) 1,3-butylene glycol | 5.0 |
| (8) Fine particle titanium oxide | 10.0 |
| (9) 2-ethyl hexyl p-methoxy cinnamate | 1.0 |
| (10) 5-(4-isobutoxy benzylidene)-3-(2-ethylhexyl)hydantoin | 1.0 |
| (11) Preservatives | q.s. |
| (12) Antioxidant | q.s. |
| (13) Perfume | q.s. |

<Method of preparation>

The ingredients (1)-(5), (9), (10), (12) and (13) were stirred and dissolved at 70 °C. Mixture of the ingredients (6)-(8) and (11) were dissolved at 70 °C. The later was added to the former. The system was emulsified and allowed to cool, and the base cosmetic was obtained.

EXAMPLE 11 DUAL PURPOSE FOUNDATION

| | |
|---|---|
| (1) Silicone treated titanium oxide | 9.5% |
| (2) Silicone treated mica | 40.0 |
| (3) Silicone treated talc | 20.45 |
| (4) Silicone treated iron oxide | 7.5 |
| (5) Spherical nylon powder | 10.0 |
| (6) Trimethylolpropanetriisostearate | 5.0 |
| (7) Squalane | 3.0 |
| (8) Bees wax | 2.0 |

| | |
|---|---|
| (9) 5-[(4-(2-ethylhexyloxy)benzylidene]-3-(2-ethylhexyl)hydantoin | 0.5 |
| (10) Sorbitan trioleate | 1.0 |
| (11) Preservatives | 0.5 |
| (12) Vitamin E | 0.05 |
| (13) Perfume | 0.5 |

<Method of preparation>

The ingredients (1)-(5) were mixed with a henchel mixer and the ingredients (6)-(13) were added therein. The product was pulverized and formed in an inner plate.

EXAMPLE 12 SUNSCREEN LOTION

| | |
|---|---|
| (1) Dimethylpolysiloxane (5cs) | 3.0% |
| (2) Methyl phenyl polysiloxane (20cs) | 17.0 |
| (3) Stearic acid | 1.0 |
| (4) 2-ethylhexyl p-dimethylaminobenzoate | 5.0 |
| (5) 5-(4-methoxybenzylidene)-3-(2-ethylhexyl)hydantoin | 10.0 |
| (6) Preservatives | 0.2 |
| (7) Perfume | 0.2 |
| (8) Glycerol | 5.0 |
| (9) Montmorillonite | 0.5 |
| (10) Potassium hydroxide | 0.2 |
| (11) Purified water | 65.9 |

<Method of preparation>

An oil part was prepared by hand-stirring the ingredients (1)-(7) at 70 °C. An aqueous part was prepared by stirring the ingredients (8)-(11) at 70 °C. The oil part was added to the aqueous part and emulsified with an emulsifier. The emulsion was allowed to cool to 30 °C with a heat exchanger and the sunscreen lotion was obtained.

As described above, the benzylidene hydantoin derivative according to the present invention can absorb ultraviolet rays in the UV-A area, and has excellent stability when the derivative is with metal ion. Furthermore, the derivative has excellent solubility in oily base.

Also. the external preparation for skin according to the present invention has excellent ultraviolet ray absorptivity and usability.

## Claims

1. A benzylidene hydantoin derivative represented by the following formula;

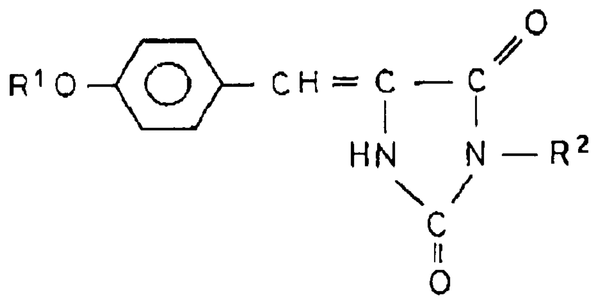

wherein $R^1$ represents a straight or branched alkyl group having 1-8 carbon atoms and $R^2$ represents a branched alkyl group having 5-18 carbon atoms.

2. An ultraviolet ray absorbent comprised of the benzylidene hydantoin derivative according to Claim 1.

3. An external preparation for skin comprising the benzylidene hydantoin derivative according to Claim 1.

European Patent Office

# EUROPEAN SEARCH REPORT

Application Number
EP 94 30 4735

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | PATENT ABSTRACTS OF JAPAN vol. 18, no. 191 (C-1186)4 April 1994 & JP-A-05 345 713 (KAO CORP) 27 December 1993 * abstract * | 1-3 | C07D233/96<br>A61K31/415<br>A61K7/42 |
| P,Y | CHEMICAL ABSTRACTS, vol. 119, no. 24, 13 December 1993, Columbus, Ohio, US; abstract no. 256290z, OKAZAKI T ET AL 'Benzylidenehydantoins and UV-absorbing skin preparations containing them' page 447 ;column 2 ; * abstract * & JP-A-05 186 429 (SHISEIDO CO LTD;JAPAN ) 27 July 1993 | 1-3 | |
| P,Y | CHEMICAL ABSTRACTS, vol. 119, no. 22, 29 November 1993, Columbus, Ohio, US; abstract no. 233707b, OKAZAKI T ET AL 'Benzylidenehydantoins and UV-absorbing skin preparations containing them' page 537 ;column 1 ; * abstract * & JP-A-05 186 430 (SHISEIDO CO LTD;JAPAN ) 27 July 1993 | 1-3 | TECHNICAL FIELDS SEARCHED (Int.Cl.6)<br>C07D<br>A61K |
| D,Y | CHEMICAL ABSTRACTS, vol. 114, no. 6, 11 February 1991, Columbus, Ohio, US; abstract no. 049354e, KOBAYASHI S ET AL 'Sunscreens containing imidazolidine derivatives' page 407 ;column 2 ; * abstract * & JP-A-02 019 314 (AJINOMOTO CO., INC.;JAPAN ) 23 January 1990 | 1-3 | |
| | -/-- | | |

The present search report has been drawn up for all claims

| Place of search | Date of completion of the search | Examiner |
|---|---|---|
| MUNICH | 29 August 1994 | Hartrampf, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 30 4735

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.6) |
| D,Y | CHEMICAL ABSTRACTS, vol. 113, no. 16, 15 October 1990, Columbus, Ohio, US; abstract no. 142414p, KATAOKA Y ET AL '5-Benzylidene-3-alkylhydantoins and additives for thermal recording materials or UV absorbers containing them' page 708 ;column 1 ; * abstract * & JP-A-02 111 760 (SHOWA DENKO K. K.;JAPAN ) 24 April 1990 --- | 1-3 | |
| A | CHEMICAL ABSTRACTS, vol. 067, no. 7, 14 August 1967, Columbus, Ohio, US; abstract no. 032646q, SHIRAI H ET AL 'Syntheses and antibacterial activity of thiohydantoin-related compounds. XII. Syntheses of alkyl compounds of 5-benzylidene-2-thiohydantoins and 2-substituted-5-benzylidenehydant oin compounds' page 3090 ;column 1 ; * abstract * & YAKUGAKU ZASSHI 1967; VOL.87 (2); PP.142-147 CITY UNIV.;FAC. PHARM. SCI.; NAGOYA; JAPAN ----- | 1-3 | TECHNICAL FIELDS SEARCHED (Int.Cl.6) |

The present search report has been drawn up for all claims

| Place of search | Date of completion of the search | Examiner |
|---|---|---|
| MUNICH | 29 August 1994 | Hartrampf, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)